# EUROPÄISCHE PATENTSCHRIFT

(11) **EP 3 348 185 B1**
(45) Veröffentlichungstag und Bekanntmachung des Hinweises auf die Patenterteilung: **11.11.2020**
(21) Anmeldenummer: 18151004.1
(22) Anmeldetag: 10.01.2018
(51) Int. Cl.: A61B 5/00, A61B 5/103, A43B 3/00, A43B 17/00

(54) **VORRICHTUNG ZUR VERMEIDUNG VON ÜBERMÄSSIGEN BELASTUNGEN AUF DEN MENSCHLICHEN FUSS BEIM GEHEN SOWIE BETRIEBSVERFAHREN HIERFÜR**
DEVICE FOR PREVENTING EXCESSIVE LOADS ON THE HUMAN FOOT DURING WALKING AND METHOD FOR THE OPERATION THEREOF
DISPOSITIF PERMETTANT D'ÉVITER DES APPUIS EXCESSIFS SUR LE PIED HUMAIN LORS DE LA MARCHE AINSI QUE PROCÉDÉ DE FONCTIONNEMENT CORRESPONDANT

(30) Priorität: 13.01.2017 DE 102017100636
(43) Veröffentlichungstag der Anmeldung: 18.07.2018
(73) Patentinhaber: Döll, Walter, 3122 Kehrsatz (CH)
(72) Erfinder: Döll, Walter, 3122 Kehrsatz (CH)
(74) Vertreter: 2K Patentanwälte Blasberg Kewitz & Reichel

(56) Entgegenhaltungen:
- CN-U- 205 080 396
- US-A1- 2004 173 220
- US-A1- 2008 216 593
- US-A1- 2014 260 677
- US-B2- 6 978 684

## Beschreibung

### Gebiet der Erfindung

Die vorliegende Erfindung betrifft allgemein die Vermeidung von übermäßigen Belastungen auf den menschlichen Fuß beim Gehen, insbesondere im Bereich der Rehabilitation oder Rekonvaleszenz nach Operationen oder im Bereich der Orthopädie oder Physiotherapie, und betrifft insbesondere die Bereitstellung eines Feedbacksignals durch die Messung von Belastungen und/oder Teilbelastungen eines Fußes, die Auswertung der Messung und Ausgabe eines Feedbacksignals sowie ein Betriebsverfahren derselben.

### Hintergrund der Erfindung

In Folge des durch den medizinischen Fortschritt bedingten gehobenen Alters und/oder durch erhöhte direkte Belastungen, wie z.B. durch Sport oder Arbeit, werden heutzutage die verschiedenen Gelenkpartien des menschlichen Körpers im Durchschnitt immer intensiver beansprucht. Insbesondere die vielbenutzten Gelenkpartien des Trage-Apparats des menschlichen Körpers, wie z.B. die Hüfte und die Knie, sind hier zu nennen. In vielen Fällen ist das jeweilige Gelenk derart beschädigt, dass eine entsprechende Operation unabdingbar wird. Hierdurch bedingt ist die Zahl der Hüft- und Knie-Operationen in den letzten Jahrzehnten drastisch angestiegen und die genannten Operationen sind zu einem Standard in der medizinischen Praxis geworden, bei denen eine natürliche Gelenkkugel durch eine Prothese ersetzt wird.

Der Patient erfährt in der Regel nach einer solchen Operation zunächst eine umfassende physiotherapeutische Unterweisung, ist aber nach dem Verlassen der Klinik weitgehend auf sich alleine gestellt. Insbesondere falsche, d.h. zu hohe, Belastungen durch den Patienten können hierbei Schäden an der Prothese oder an dem umgebenden Gewebe entstehen lassen. Um dies zu verhindern wurden verschiedene Systeme entwickelt, um die Belastung des Fußes des Patienten zu überwachen.

US 2016 158 622 A1 offenbart ein herkömmliches Gehtrainingssystem ("Walking Training System") mit einem Laufband und zwei Rahmen von denen aus der Patient unterstützt wird, wobei Sensoren die Gewichtsverteilung beim Laufen messen. Dieses System ist ein klassisches System, das in der Physiotherapie verwendet wird. Für den privaten Gebrauch ist es aber zu teuer (da z.B. ein Laufband verwendet wird), zu sperrig und zu unpraktisch (insbesondere für den Alltagsgebrauch).

US 4 746 930 offenbart eine Vorrichtung, welche auf einer relativ schmalen Schuhsole integriert ist und Sensor-Einheiten umfasst, die in dafür vorgesehenen Kammern vorgesehen sind und Paare von elektrischen Kontakten umfasst, die abwechselnd, fingerförmig ausgeprägt sind und durch Druck aufeinandergedrückt werden können, um einen elektrischen Kontakt zu erzeugen. Leider ist diese Vorrichtung jedoch im Normalfall über ein Kabel mit einem Unterstützungsrahmen verbunden, um Signale an diesen zu übertragen und umfasst zudem ein anfälliges Sensoren-System, das in die Schuhsohle integriert ist und keinerlei Auslesemöglichkeiten bietet. Hierdurch wird ein praktischer Alltagsgebrauch weitestgehend unmöglich gemacht.

US 2005 026 160 A1 offenbart eine einfach aufgebaute Vorrichtung, welche in einer relativ breiten Schuhsole integriert ist, und einen Zustands-Sensor umfasst, der den physischen Zustand des Fußes misst, sowie einen RF-Sender, der gemäß der Messung ein Signal an eine externe Display-Einheit sendet. Die genannten Zustands-Sensoren werden hierbei in dafür vorgesehenen Installations-Nischen angebracht. Obwohl die genannte Vorrichtung in der Schuhsohle integriert ist, entstehen dennoch durch die erhebliche Breite der Schuhsohle Unannehmlichkeiten. Außerdem werden die Messungs-Signale durch einen RF-Sender übertragen, was zum einen eine externe Empfangseinheit (z.B. einem Display-Armband oder einem Smartphone) und zum anderen einen ununterbrochenen Übertragungsweg zwischen dem RF-Sender und der Empfangseinheit notwendig macht.

US 5 033 291 offenbart einen flexiblen Sensor, der in der Lage ist Druckverteilungen des Fußes beim Auftreten zu messen, wobei dieser Sensor eine Vielzahl von flexiblen Elektroden umfasst, die in zwei nicht parallel liegende Sets unterteilt sind, um die genannten Druckverteilungen zu messen. Insbesondere ist der Sensor bevorzugter weise in Fuß Form ausgelegt. Ein ähnliches flexibles Sensorsystem wird des Weiteren auch in US 5 408 873 offenbart, wobei in dieser Erfindung auch mehrere Kraftsensoreinheiten an den essentiellen Messungs-Stellen platziert sein können. Die genannten Sensoren sind jedoch in Ihrer offenbarten Form relativ kompliziert aufgebaut und teuer. Des Weiteren ist in den genannten Offenbarungen kein Feedback-System vorgesehen.

Zusätzlich zu den genannten Vorrichtungen und Systemen aus dem medizinischen Bereich existieren des Weiteren unzählige Systeme, die Verwendung im Sportbereich finden, wobei diese meist lediglich zum direkten Messen von Trainingsparameter, wie z.B. der Kontaktzeit der Füße und der Schrittfrequenz verwendet werden. Ein solches System wird z.B. in der amerikanische Patentschrift US 6 876 947 B1 offenbart, die eine am Schuh befestigte Einheit umfasst, die mittels Funk, gewonnene Mess-Signale, wie z.B. die Kontaktzeit des Fußes, an eine Anzeige-Einheit (z.B. Smart-Phone oder Smart-Watch) sendet. Auch bei dieser Offenbarung ergeben sich die zuvor genannten Probleme, die bei der Funkübertragung auftreten können, und das Problem des fehlenden (direkten) Feedbacks.

US 2014/260677 A1 offenbart eine Vorrichtung gemäß dem Oberbegriff von Patentanspruch 1 zum Auswerten von Fitnessaktivitäten. Hierzu nehmen Kraftsensoren an mehreren Positionen im Bereich einer Schuhsohle Kräfte auf, die dann ausgewertet werden. Entsprechend der Lehre nach der in Bezug genommenen US 6 978 684 B2 wird dann ein taktiles Feedback mit Hilfe von Vibrationselementen erzeugt. US 2014/260677 A1 offenbart hierzu zwei Ausführungsformen, nämlich die Anordnung der Elektronik auf einem Substrat, das in eine Schuhsohle beispielsweise eines Laufschuhs integriert ist, oder die Anordnung der Elektronik in einer Schuheinlage. Nach der ersten Ausführungsform kann die Elektronik in einer Art Gehäuse angeordnet sein, die ihrerseits in einer Aufnahme innerhalb der Schuhsohle angeordnet ist. Das taktile Feedback wird jedoch nicht spezifisch im Bereich des Fuß-Längsgewölbes des Benutzers erzeugt.

CN 205 080 396 U offenbart die Integration einer Energiequelle gemeinsam mit anderen elektronischen Komponenten in ein Gehäuse in einer Schuheinlage.

US 2008/216593 A1 offenbart die Erkennung eine Gangart und Ausgabe eines FeedbackSignals, wenn die Gangart nicht passend ist. Hierzu ist ein Vibrationsalarm angedacht. Die zugehörige Elektronik offenbar in eine Schuheinlage integriert werden.

US 2004/173220 A1 offenbart ein System zur Verhinderung von Fußverletzungen durch neurologische Stimulation des Fusses and Knöchels, Hierzu werden diverse Ausführungsformen offenbart, bei denen jedoch kein Feedback spezifisch im Bereich des Fuß-Längsgewölbes angewendet wird.

### Beschreibung der Erfindung

Es ist die Aufgabe der vorliegenden Erfindung, eine kostengünstige und alltagstaugliche Vorrichtung bereitzustellen, die Belastungen bzw. Teilbelastungen eines Fußes messen und auswerten kann, um dem Benutzer in einfacher Weise ein direktes Feedback bereit stellen zu können, um übermäßige Belastungen auf den menschlichen Fuß beim Gehen zu vermeiden.

Gelöst wird die Aufgabe durch die Vorrichtung nach Anspruch 1 und dem Betriebsverfahren nach Anspruch 15. Weitere vorteilhafte Ausführungsformen sind Gegenstand der rückbezogenen Unteransprüche.

Gemäß der vorliegenden Erfindung wird eine Vorrichtung zur Vermeidung von übermäßigen Belastungen eines menschlichen Fußes beim Gehen bereitgestellt. Die Vorrichtung umfasst eine Schuheinlage mit mindestens einem Kraftsensor, eine Auswertungseinheit, eine elektrische Energiequelle, einen Feedback-Steuerungssignal-Generator und eine Feedback-Ausgabe-Einheit. Dabei ist der mindestens eine Kraftsensor auf oder in der Schuheinlage angeordnet, um ein Messsignal zu erzeugen, das Belastungen und/oder Teilbelastungen des Fußes anzeigt. Die Auswertungseinheit ist elektrisch leitend mit dem mindestens einen Kraftsensor verbunden und ausgelegt, um das Messsignal von dem mindestens einen Kraftsensor auszuwerten. Die elektrische Energiequelle ist ausgelegt, um Strom für die Auswertungseinheit bereitzustellen. Der Feedback-Steuerungssignal-Generator ist mit mindestens einer zugeordneten Feedback-Ausgabe-Einheit verbunden und ausgelegt, um auf der Grundlage der Auswertung der Auswertungseinheit ein Feedback-Steuerungssignal zu erzeugen, durch welches die Feedback-Ausgabe-Einheit angesteuert wird, um ein Feedbacksignal im Bereich des Fußes auszugeben. Dabei ist die Feedback-Ausgabe-Einheit als Aktuator ausgebildet, der das Feedback-Steuerungssignal erhält und ausgelegt ist, um ein taktiles Feedback im Bereich des Fuß-Längsgewölbes auszugeben.

Erfindungsgemäß sind die Auswertungseinheit, die Energiequelle und der Feedback-Steuerungssignal-Generator als gemeinsame Einheit in einem Hohlraum eines Gehäuses angeordnet sind, das in die Schuheinlage im Bereich seines Fuß-Längsgewölbes integriert ist, wobei das Gehäuse gewölbt und korrespondierend zu einer durchschnittlichen Form eines Fuß-Längsgewölbes ausgebildet ist, um einen Fuß im Bereich des Fuß-Längsgewölbes unmittelbar abzustützen, und wobei der Aktuator auf dem oder unmittelbar an dem Gehäuse oder in dem Gehäuse angeordnet ist, um das taktile Feedback im Bereich des Fuß-Längsgewölbes auszugeben.

Dadurch kann die Vorrichtung auf der Basis von gemessenen Belastungen und/oder Teilbelastungen des Fußes, also von Belastungen und/oder Teilbelastungen, die auf den Fuß einwirken, ein sogenanntes Bio-Feedback im Bereich des Fußes erzeugen.

Die Schuheinlage gemäß der vorliegenden Erfindung ist zweckmäßig möglichst flach gehalten, um in verschiedenen Schuhen als Ersatz für eine reguläre Schuheinlage angeordnet werden zu können. Die einzelnen Elemente, wie z.B. die Kraftsensoren oder die Leiterbahnen zur elektrischen Verbindung der Bauelemente, können entweder nicht lösbar auf der Schuheinlage befestigt sein (z.B. durch Verkleben, Einlaminieren, Eingießen, Einschäumen etc.), oder aber an dieser befestigt sein (z.B. durch Anclipsen), so dass sowohl Ausführungen der vorliegenden Erfindung denkbar sind, bei denen die Vorrichtung mehrmals wiederverwendet wird, z.B. als Installation in Laufschuhen, oder aber nur wenige Male zur Anwendung kommt, z.B. im Krankenhausbetrieb. Insbesondere lässt sich die Schuheinlage sowohl in herkömmlichen Schuhen als auch in medizinischen, schuhähnlichen Vorrichtungen anordnen.

Die verwendeten Kraftsensoren können in verschiedenen Ausführungen vorliegen. Zweckmäßig sind die Kraftsensoren zum einen möglichst flach ausgebildet und zum anderen auch dehnbar bzw. flexibel ausgebildet, um sich an die jeweilige Fußsohle anpassen zu können. Insbesondere Kraftsensoren, die auf der Basis von Foliensensoren basieren, bieten sich für die genannte Aufgabe an, um Belastungen und/oder Teilbelastungen des menschlichen Fußes beim Gehen zu messen. Hierbei wird ein Foliensensor durch das Aufbringen eines Druckes eines bestimmten Teils des Fußes entsprechend verformt. Auch andere Kraftsensoren, insbesondere jedoch kapazitive Kraftsensoren, die mit einem verformbaren Dielektrikum gefüllt sind und beim Anliegen eine Kraft von oben ihre Kapazität ändern, sind im Sinne der Erfindung anwendbar.

Die elektrischen Leiterbahnen können gemäß der vorliegenden Erfindung in unterschiedlichen Ausführungen vorliegen. Insbesondere können diese in kabelähnlichen Strukturen auf die Schuheinlage aufgeklebt sein oder in diese eingearbeitet sein. Jedoch ist auch ein direktes aufbringen der Leiterbahnen auf der Schuhsohle durch ein "electric-circuit-printing"-Verfahren möglich.

Die elektrische Energiequelle ist gemäß der vorliegenden Erfindung bevorzugter Weise eine handelsübliche Knopfzelle (als Primärelement), wobei jedoch auch wieder aufladbare Zellen möglich sind. Insbesondere sind Knopfzellen mit Primärelementen bei temporär benutzten Varianten der vorliegenden Erfindung von Interesse, z.B. in der Rehabilitation nach einer Operation, wohingegen Akkus oder auswechselbare Batterien bei dauerhaft benutzbaren Varianten auch eine Rolle spielen. Außerdem sind auch zusätzliche Miniatur-Generatoren möglich, um die elektrische Energiequelle nachzuladen (wie z.B. beim "energy harvesting") oder Kombinationen der zuvor genannten Beispiele.

Der Feedback-Steuerungssignal-Generator erzeugt, wie schon zuvor erwähnt wurde, ein Feedback-Steuerungssignal, wobei dieses Feedback-Steuerungssignal genutzt wird, um verschiedene Anzeige-Mittel anzusteuern. Insbesondere ist hier die Ansteuerung von Aktuatoren zu nennen, wobei ergänzend aber auch weitere Anzeige-Mittel (z.B. optische oder akustische) vorgesehen sein können. Es sei an dieser Stelle darauf verwiesen, dass der Feedback-Steuerungssignal-Generator sowohl als einzelne Einheit vorliegen kann, als auch in der Auswertungseinheit integriert sein kann. Die zweite Variante ist insbesondere bei leistungsstärkeren Auswertungseinheiten, wie z.B. MCUs oder gar CPUs, bevorzugt.

Erfindungsgemäß ist der Aktuator ausgelegt, um als taktiles Feedback Vibrationen auf dem Gehäuse oder in dessen unmittelbarer Nähe zu erzeugen. Diese wirken im Bereich des Fuß-Längsgewölbes und damit in einem besonders sensiblen Bereich auf den menschlichen Fuß ein, sodass auch Vibrationen mit vergleichsweise geringer Intensität, die energiesparend ohne weiteres erzeugt werden können, ausreichend sein können, um der menschlichen Person ein Feedback zum Gehverhalten zu geben. Dieses Feedback wird zweckmäßig so erzeugt, dass beim Gehen in einem akzeptablen Bereich, in dem die Belastungen und/oder Teilbelastungen auf den menschlichen Fuß in einem akzeptablen Bereich liegen, kein Feedback ausgegeben wird, während in einem Bereich, in dem die Belastungen und/oder Teilbelastungen auf den menschlichen Fuß nicht mehr in einem akzeptablen Bereich liegen, beispielsweise weil der Fuß zu sehr seitlich oder verkantet aufsetzt, ein taktiles Feedback ausgegeben wird, das den Patienten unmittelbar warnt und somit dazu auffordert, wieder in eine akzeptablen Gehbereich zurückzukehren. Das Feedback kann dabei als kontinuierliches oder intermittierendes taktiles Feedback-Signal ausgegeben werden.

In einer besonders bevorzugten Ausführungsform umfasst die Vorrichtung des Weiteren ein Gehäuse, welches in die Schuheinlage integriert ist und eine elektrische Leiterplatte umfasst. Das Gehäuse ist zweckmäßig ausreichend abgedichtet, um das Eindringen von Feuchtigkeit oder gar Nässe in den Innenraum des Gehäuses zuverlässig zu verhindern. Dies kann mittels Dichtungen oder geeignete Ausbildung von Gehäuseschalen in einfacher Weise bewerkstelligt werden.

Dabei kann das Gehäuse die Auswertungseinheit, die elektrische Energiequelle und den Feedback-Steuerungssignal-Generator umfassen/aufnehmen und die Feedback-Ausgabe-Einheit, um diese zu schützen. Insbesondere der Schutz gegen Wasser, z.B. in Form von Schweiß oder in den Schuh eindringendes Regenwasser, und der Schutz gegen hohe Druckbelastungen, die z.B. durch das Aufsetzen des Fußes entstehen können, sind hier zu erwähnen. Das Gehäuse ist vorzugsweise im Bereich eines Fuß-Längsgewölbes vorgesehen, da hier zum einen ausreichend Platz zur Befestigung zur Verfügung steht und zum anderen auch zusätzlicher Schutz besteht, da die Hauptdruckpunkte des Fußes normalerweise im Ballen- und/oder Fersenbereich und nicht im Bereich des Fuß-Längsgewölbes liegen. Außerdem ist dieser Bereich leicht stimulierbar und daher gut für taktile Signale zugänglich. Des Weiteren sind natürlich auch Anordnungen denkbar, in denen ein verstärktes Gehäuse auch im Fersen- oder Ballenbereich angeordnet ist. Weiterhin ist auch ein nicht abgedichtetes Gehäuse möglich wobei das Gehäuse hierbei geöffnet werden kann, etwa durch Lösen einer Clips- oder Schraubverbindung. Diese Ausführungsform ist insbesondere im klinischen Bereich, insbesondere jedoch in der Physiotherapie denkbar.

Zusätzlich zu diesem zentralen Gehäuse sind jedoch auch mehrere Teilgehäuse möglich, um einzelne sensible Komponenten zu schützen.

In einer bevorzugten Ausführungsform umfasst die Vorrichtung des Weiteren eine I/O-Schnittstelle (Eingabe-/Ausgabe-Schnittstelle). Mit Hilfe dieser I/O-Schnittstelle können die Auswertungseinheit sowie andere mögliche Komponenten programmiert werden, wobei hier zum einen die Einspielung einer bestimmten "embedded Firmware" möglich ist, oder aber das einfache Setzen bestimmter Einstell-Parameter der Vorrichtung, wie z.B. eine Sensibilität der Kraftsensoren oder eine Feedbackstärke. Außerdem ist auch ein Auslesen der Auswertungseinheit möglich, um z.B. Daten auf einem Computer oder Smartphone zu analysieren. Die I/O-Schnittstelle kann als leitungsgebundene Schnittstelle mit lösbarer Steckverbindung, z.B. als USB-Anschluss, vorliegen oder in bevorzugter Weise als kabellose Schnittstelle. Bei einer kabellosen Schnittstelle bietet sich insbesondere eine "Near Field Communication" NFC-Schnittstelle an, bei der eine Übertragungsspule in dem abgedichteten Gehäuse der Vorrichtung angeordnet ist und in einfacher Weise durch ein geeignetes Gerät, wie z.B. ein Smartphone, ausgelesen werden kann. Die vorgesehene NFC-Schnittstelle arbeitet wechselseitig nach dem Prinzip der elektromagnetischen Induktion. Hierzu wird eine möglichst große Induktionsspule innerhalb der Einlagesohle oder des abgedichteten Gehäuses verlegt. In einer zweiten Funktion dient die Induktionsspule auch zum kontaktlosen Laden der elektrischen Energiequelle, falls diese wieder aufladbar ist. Hierzu ist eine spezielle Ladevorrichtung, die die elektrische Energiequelle über Nacht nach dem Prinzip der elektromagnetischen Induktion auflädt, erforderlich. Diese Ladevorrichtung kann z.B. als Fußmatte mit eingelassenen Induktionsspulen zur magnetischen Felderzeugung ausgelegt sein.

In einer bevorzugten Ausführungsform umfasst die Vorrichtung des Weiteren ein Speicherelement. Auf diesem Speicherelement können Auswertungsdaten der Auswertungseinheit gespeichert werden, um z.B. später analysiert zu werden. Des Weiteren können auch Programme auf dieser Einheit abgespeichert sein. Das Speicherelement kann entweder fest eingebaut sein (embedded chip) oder herausnehmbar sein (z.B. eine flash card).

In einer besonders bevorzugten Ausführungsform der vorliegenden Erfindung umfasst die Vorrichtung mindestens einen Aktuator als Feedback-Ausgabe-Einheit, der das Feedback-Steuerungssignal des Feedback-Steuerungssignal-Generators erhält und ein taktiles Feedback im Bereich der Schuheinlage erzeugt. Hierbei kann die Stärke des taktilen Feedbacks angepasst werden, um dem Benutzer ein angemessenes Feedback zur Verfügung zu stellen. Für die Ausführung des Aktuators sind verschiedene Varianten möglich, wobei die Aktuatoren sowohl als rotierende Kleinstmotoren mit Exzenter (analog in der Funktion wie ein Vibrations-Alarm in einem Mobiltelefon) als auch als Vibratoren mit ausschließlich translatorischer Bewegungsrichtung angeordnet sein können. Alternativ kann der Aktuator auch analog zu einem Lautsprecher aufgebaut sein, wobei eine Erreger-Spule fest mit dem oberen Teil des abgedichteten Gehäuses verbunden ist, während ein Permanentmagnet zusammen mit einem weichmagnetischen Führungsmaterial zur Führung des magnetischen Flusses mit dem unteren Teil des abgedichteten Gehäuses verbunden ist. Wenn ein sich ändernder Strom durch die Erregerspule fließt, so üben Erregerspule und Permanentmagnet eine Kraft aufeinander aus, die auf das Längsgewölbe übertragen wird.

In einer Ausführungsform der vorliegenden Erfindung umfasst die Vorrichtung eine Mehrzahl von Kraftsensoren und eine Mehrzahl von Aktuatoren, die verteilt in der Schuheinlage angeordnet sind. Die Aktuatoren sind in dieser Ausführungsform in der Nähe eines jeweiligen Kraftsensors angeordnet, um an mehreren Positionen der Schuheinlage ein taktiles Feedback zu erzeugen, sodass die Feedback-Ausgabe auf der Basis der Teilbelastungen des Fußes an den mehreren Positionen der Schuheinlage erfolgen kann. Diese Ausführungsform spielt insbesondere im sportmedizinischen Bereich und zu Trainingszwecken eine Rolle, da hierdurch in einfacher Weise falsche Lauf- bzw. Belastungs-Muster abtrainiert werden können, um somit verschiedensten Schädigungen vorzubeugen, wie z.B. Achillessehnen-Zerrungen, Fußgelenks-Über- und Falschbelastungen oder sogar Knie-, Hüft- und Wirbelsäulenschädigungen. Des Weiteren kann der bzw. die aktuatorische/n Signalgeber auch gleichzeitig mit anderen Signalgebern (z.B. akustischen oder optischen Signalgebern) verwendet werden.

Die genannte Ausführungsform lässt sich jedoch auch weitläufig im Trainingsbereich verwenden, um verschiedene Fußbelastungen bei bestimmten Sportarten zu trainieren, wie z.B. dem Ballenlauf oder dem Außenspannlauf.

In einer weiteren Ausführungsform der vorliegenden Erfindung umfasst die Vorrichtung des Weiteren (mindestens) einen akustischen Signalgeber der als zusätzliche Feedback-Ausgabe-Einheit dient und der das Feedback-Steuerungssignal des Feedback-Steuerungssignal-Generators erhält und ein akustisches Feedback ausgibt. Der akustischen Signalgeber kann insbesondere ein am Außenbereich der Sohle befestigter Lautsprecher sein, der in Entsprechung zu dem Feedback-Steuerungssignal, z.B. Digitaltöne, ausgibt. Der akustischen Signalgeber muss jedoch hörbar angebracht sein, so dass der Benutzer die akustischen Signale wahrnehmen kann. Insbesondere kann der akustische Signalgeber auch gleichzeitig mit anderen Signalgebern verwendet werden.

In einer Ausführungsform der vorliegenden Erfindung umfasst die Vorrichtung mindestens ein Leuchtelement als zusätzliche Feedback-Ausgabe-Einheit, das das Feedback-Steuerungssignal des Feedback-Steuerungssignal-Generators erhält und ein Leuchtsignal ausgibt. Das Leuchtelement muss passend im Außenbereich des Schuhs angeordnet sein, damit der Benutzer die Leuchtsignale sehen kann. Insbesondere kann der optische Signalgeber auch gleichzeitig mit anderen Signalgebern verwendet werden.

In einer bevorzugten Ausführungsform der vorliegenden Erfindung umfasst die Vorrichtung des Weiteren eine Übertragungs-Einheit, um direkt Daten und/oder Signale der Auswertungseinheit oder das/die Feedback-Steuerungssignal/e des Feedback-Steuerungssignal-Generators an eine externe Anzeige-Einheit zu übertragen. Die Übertragungs-Einheit kann insbesondere als ein Funksensor oder als ein Kabel vorliegen, wobei der Funksensor bevorzugt an der Außenseite des Schuhs, z.B. an den Schnürsenkeln, an der Außenseite des Schuhs oder am Beingelenk, befestigt ist. Die Übertragungs-Einheit der vorliegenden Erfindung ist eine optionale Einheit, die dem Benutzer eine zusätzliche Kontrolle, z.B. auf einem Display ermöglicht.

In einer besonders bevorzugten Ausführungsform der vorliegenden Erfindung ist die Vorrichtung in einem Schuh oder einem schuhähnlichen medizinischen Gestell angeordnet, wobei hier sowohl eine permanente Anbringung der Schuheinlage, z.B. durch Verkleben, als auch eine herausnehmbare Schuheinlage möglich sind.

In einer bevorzugten Ausführungsform der vorliegenden Erfindung umfasst die Erfindung des Weiteren ein Verfahren zum Betreiben einer Vorrichtung, wie vorstehend dargelegt, das mindestens die folgenden Schritte umfasst: Messen von Belastungen und/oder Teilbelastungen mit dem mindestens einen Kraftsensor sowie Erzeugen eines Messsignals, das die Belastungen und/oder Teilbelastungen des Fußes anzeigt; Auswerten des durch die Auswertungseinheit empfangenen Messsignals und Erzeugen eines Feedback-Steuerungssignals auf der Grundlage der Auswertung durch die Auswertungseinheit und Steuern einer Feedback-Ausgabe-Einheit auf der Grundlage des Feedback-Steuerungssignals; und Ausgeben eines Feedbacks über die Feedback-Ausgabe-Einheit auf der Basis des Feedback-Steuerungssignals.

In einer weiteren besonders bevorzugten Ausführungsform des Verfahrens der vorliegenden Erfindung umfasst das Verfahren das Übermitteln des Feedback-Steuerungssignals an zumindest eine Feedback-Ausgabe-Einheit, die ein Aktuator, ein optischer oder ein akustischer Signalgeber ist, um dementsprechend jeweils ein taktiles Feedback, ein optisches Feedback oder ein akustisches Feedback zu erzeugen und damit eine Überwachung von Belastungen und/oder Teilbelastungen des Fußes im Bereich der Schuheinlage zu ermöglichen. Wie schon zuvor erwähnt wird das taktile Feedback hierbei im Bereich der Schuheinlage ausgegeben.

Es sei an dieser Stelle darauf hingewiesen, dass die beschriebenen Ausführungsformen auch untereinander kombiniert werden können, ohne dabei den Schutzumfang der vorliegenden Erfindung einzuschränken.

### Kurze Beschreibung der Zeichnungen

Im Folgenden werden die Zeichnungen der bevorzugten Ausführungsformen kurz beschrieben.
**Fig. 1** zeigt eine schematische Ansicht einer Schuheinlage, mit der Vorrichtung einer bevorzugten Ausführungsform der vorliegenden Erfindung.
**Fig. 2** zeigt eine schematische Schnitt-Ansicht durch das abgedichtete Gehäuse der Vorrichtung in einer besonders bevorzugten Ausführungsform der vorliegenden Erfindung.
**Fig. 3** zeigt eine schematische Schnitt-Ansicht eines taktilen Aktuators der Vorrichtung in einer besonders bevorzugten Ausführungsform der vorliegenden Erfindung.
**Fig. 4a** zeigt eine schematische Ansicht einer Schuheinlage, mit der Vorrichtung einer weiteren bevorzugten Ausführungsform der vorliegenden Erfindung bei der zusätzlich ein akustischer Signalgeber vorliegt.
**Fig. 4b** zeigt eine schematische Ansicht einer Schuheinlage, mit der Vorrichtung nach einer Ausführungsform der vorliegenden Erfindung bei der zusätzlich ein Leuchtelement vorliegt.
**Fig. 5** zeigt ein Blockschaltbild einer besonders bevorzugten Ausführungsform der vorliegenden Erfindung.
**Fig. 6** zeigt eine schematische Ansicht einer Schuheinlage, mit der Vorrichtung nach noch einer bevorzugten Ausführungsform der vorliegenden Erfindung bei der des Weiteren eine zusätzliche Übertragungs-Einheit vorliegt.

### Ausführliche Beschreibung der Zeichnungen

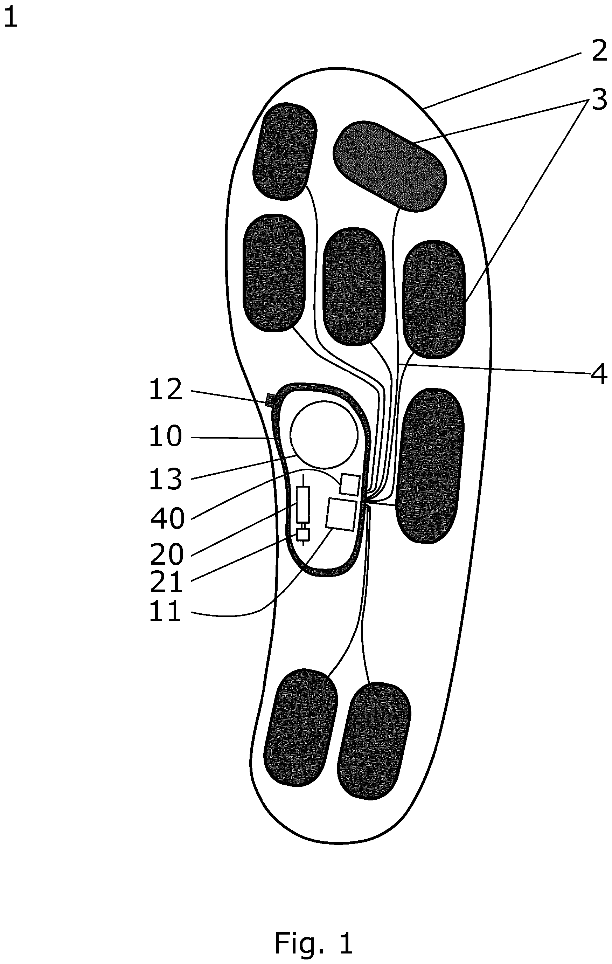

In Fig. 1 ist eine Vorrichtung 1 zur Vermeidung von übermäßigen Belastungen eines menschlichen Fußes beim Gehen gemäß einer besonders bevorzugten Ausführungsform dargestellt. Die Vorrichtung 1 umfasst hierbei eine Schuheinlage 2 mit mindestens einem Kraftsensor 3 zum Erzeugen eines Messsignals, das die Belastungen und/oder Teilbelastungen des Fußes anzeigt; eine Auswertungseinheit 11, die über elektrische Leitungen 4 mit dem mindestens einen Kraftsensor 3 verbunden ist, um das Messsignal von dem mindestens einen Kraftsensor 3 auszuwerten; eine elektrische Energiequelle 13, um Strom für die Auswertungseinheit 11 bereitzustellen; und des Weiteren eine Feedback-Steuerungssignal-Generator 20, die auf Grundlage der Auswertung durch die Auswertungseinheit 11 ein Feedback-Steuerungssignal erzeugt, um die Überwachung von Belastungen und/oder Teilbelastungen des Fußes zu ermöglichen. Außerdem umfasst die Vorrichtung 1 einen Aktuator 22 als Feedback-Ausgabe-Einheit 21, der das Feedback-Steuerungssignal erhält und ein taktiles Feedback im Bereich der Schuheinlage 2 erzeugt. Die sensiblen Bauteile, d.h. die Auswertungseinheit 11, die elektrische Energiequelle 13, der Feedback-Steuerungssignal-Generator 20 und der Aktuator 22 sind in der vorliegenden Ausführungsform in einem abgedichteten Gehäuse 10 angeordnet, um diese vor Wasser und/oder Druckbelastungen zu schützen. Eine Abdichtung des Gehäuses 10 ist jedoch nicht zwingend erforderlich, etwa beim Einsatz in trockenen Räumlichkeiten, beispielswiese bei der Physiotherapie.

Gemäß der Fig. 1 sind in der Schuheinlage mehrere Kraftsensoren 3 verteilt und in anatomisch unterscheidbaren Regionen angeordnet. Dargestellt ist eine erste Gruppe von Kraftsensoren 3 im Bereich der Zehen des menschlichen Fußes, insbesondere in den jeweiligen Kontaktbereich der vorderen Enden der Zehen mit der Schuheinlage 2. Dabei kommt dem großen Zeh bevorzugt eine besondere Bedeutung zu, die sich in einem eigens für diesen vorgesehenen Messbereich eines einzelnen Kraftsensors 3 wiederspiegelt. Zweckmäßig sind die weiteren Messbereiche der Kraftsensoren 3 für die kleinen Zehen wiederum zu einem eigenen Kraftsensor zusammengefasst. Alternativ können auch jedem einzelnen der kleinen Zehen eigene Kraftsensoren 3 in der vorgenannten Art zugeordnet sein. Weiterhin ist zumindest ein Kraftsensor 3 angrenzend an den Fußbogen (nicht gezeigt) im Bereich des Fußballens angeordnet. Bei dem dargestellten Ausführungsbeispiel sind im Bereich des Fußballens drei Kraftsensoren 3 angeordnet, von denen ein Kraftsensor 3 bevorzugt mittig auf der Schuheinlage 2 angeordnet ist und zwei weitere Kraftsensoren 3 jeweils links und rechts zu diesem mittigen Kraftsensor 3 angeordnet sind. Durch die beiden außermittigen Kraftsensoren 3 ist grundsätzlich die Feststellung einer seitlichen Fehlbelastung des Fußes möglich, etwa durch Verkanten oder verkipptes Auftreten des Fußes.

Weiterhin ist gemäß der Fig. 1 seitlich zum Gehäuse 10 ein weiterer Kraftsensor 3 angeordnet. Bevorzugt ist das Gehäuse 10 im Bereich des Fußlängsgewölbes angeordnet, sodass dieser einzelne Kraftsensor 3 unmittelbar neben dem Fußlängsgewölbe angeordnet ist, als in demjenigen Bereich, in dem der Fuß üblicherweise auf der Schuheinlage 2 abgestützt ist. Mit Hilfe dieses Sensors ist weiter eine Auswertung von Kraftsignalen dahingehend möglich, wie groß die Belastung im mittleren Bereich des Fußes ist und insbesondere eine mögliche Fehlbelastung durch einseitig seitliches oder verkipptes Auftreten ist.

Weiterhin ist gemäß der Fig. 1 zumindest ein Kraftsensor 3 im Bereich der Ferse des Fußes angeordnet. Bevorzugt sind im Bereich der Ferse des Fußes zwei Kraftsensoren 3 angeordnet, zweckmäßig symmetrisch oder nahezu symmetrisch zu einer gedachten Längsmittellinie der Schuheinlage. Durch diese beiden außermittigen Kraftsensoren 3 im Bereich der Ferse ist weiterhin die Feststellung einer Fehlbelastung des Fußes möglich, etwa durch zu starkes Auftreten des Fußes im Bereich der Ferse, etwa bei sog. "Fersengehern".

Bevorzugt werden die Signale der jeweiligen Messaufhehmer (also der Kraftsensoren 3) einzeln über jeweilige Leiterbahnen 4 an die Auswertungseinheit 4 weitergeleitet, die diese auch einzeln einlesen und weiterverarbeiten kann. Dadurch wird erfindungsgemäß grundsätzlich eine ortsaufgelöste Messung von Belastungen und/oder Teilbelastungen eines menschlichen Fußes möglich, um auf Grundlage einer geeigneten Auswertung der Messsignale ein Feedbacksignal auszugeben, wie nachfolgend beschrieben.

Wenn die Vorrichtung 1 zur Ausgabe des Feedbacksignals nur einen Aktuator 22 aufweist, so können die mehreren Kraft-Messsignale gemittelt werden, um ein Feedback-Steuerungssignal zu erzeugen. Diese Mittelung kann grundsätzlich über den gesamten Bereich der Schuheinlage 2 erfolgen. Optional kann die Auswertungseinheit 11 auch gesteuert oder geeignet in einen Betriebsmodus umgeschaltet werden, sodass nicht sämtliche Kraftsensoren 3, sondern nur eine Teilgruppe von Kraftsensoren 3 zur Auswertung und Erzeugung des Feedback-Steuerungssignals verwendet werden. Zweckmäßig entsprechen diese Gruppen denen in der Fig. 1 dargestellten Gruppen. Dabei können auch zwei oder mehrere Gruppen zu einer Untergruppe logisch zusammengeschaltet werden.

Wenn die Vorrichtung 1 zur Ausgabe des Feedbacksignals mehrere Aktuatoren 22 aufweist, so kann grundsätzlich die vorgenannte Mitteilung oder Durchschnittswertbildung auch angewendet werden, um ein für sämtliche Aktuatoren 22 einheitliches Feedback-Steuerungssignal zu erzeugen.

Gemäß einer weiteren Ausführungsform können in der Schuheinlage 2 mehrere Aktuatoren 22 verteilt angeordnet sein. Beispielsweise kann ein Aktuator 22 im Bereich des Fußlängsgewölbes angeordnet sein, etwas in der Art, wie in der Fig. 1 gezeigt, und kann ein weiterer Aktuator 22 im Fersenbereich und/oder ein weiterer Aktuator 22 im Zehenbereich angeordnet sein. Im letztgenannten Fall können dem jeweiligen weiteren Aktuator 22 Gruppen von Kraftsensoren 3 zugeordnet sein, die gemeinsam von der Auswertungseinheit 11 ausgewertet werden. Auf diese Weise kann grundsätzlich auch an mehreren Bereichen, bevorzugt an zwei oder drei Bereichen, der Schuheinlage 2 ein Feedbacksignal bereitgestellt werden.

In diesem Ausführungsbeispiel der vorliegenden Erfindung ist die Vorrichtung 1 mit einer I/O-Schnittstelle 12 versehen, um die Vorrichtung 1 auslesbar und programmierbar zu machen, wobei die I/O-Schnittstelle 12 insbesondere als NFC-Schnittstelle vorliegen kann und oder als Steck-Schnittstelle, wobei hierbei (mindestens) ein Ausgang derselben am äußeren Rand des abgedichteten Gehäuses 10 angeordnet ist. Durch Auslesen können beispielsweise Bewegungsprofile ausgelesen und extern weiterverarbeitet werden, was insbesondere dann von Vorteil ist, wenn in der Schuheinlage 2 mehrere Kraftsensoren 3 verteilt angeordnet sind. Durch das Programmieren kann beispielsweise die Zuordnung und/oder logische Zusammenschaltung der Kraftsensoren 3 zur Auswertung der Kraftsignale verändert werden. Dadurch kann die Vorrichtung erfindungsgemäß auf andere Nutzer rasch umprogrammiert werden.

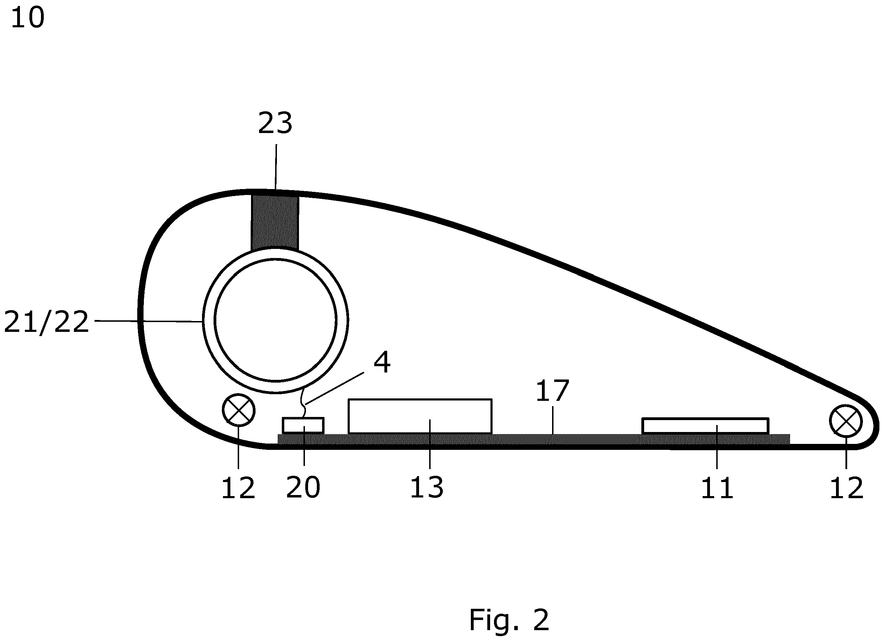

In Fig. 2 ist eine schematische Detailansicht des abgedichteten Gehäuses 10 der Vorrichtung 1 nach dem vorherigen Ausführungsbeispiel der vorliegenden Erfindung dargestellt.

Das Gehäuse 10 ist zweckmäßig ein geeignet geformtes Kunststoffgehäuse mit einem Hohlraum in dem die weiter unten genannten elektronischen Bauelemente aufgenommen sind. Das Gehäuse kann als Einweggehäuse ausgebildet sein, in dem diese elektronischen Bauelemente verkapselt oder eingegossen sind. Das Gehäuse 10 kann gemäß weiteren Ausführungsformen auch geöffnet werden, etwa durch lösen einer Clips- oder Schraubverbindung. Das Gehäuse weist weiterhin geeignete Kabeldurchführungen für die Leiterbahnen 4 auf, damit die elektrischen Signale in das Gehäuse eingespeist und ausgespeist werden können. Zweckmäßig können die Leiterbahnen 4 in das Gehäuse 10 direkt eingegossen werden, wodurch automatisch auch eine Abdichtung erzielt wird. Denkbar sind jedoch auch eigens hierzu vorgesehene Kabeldurchführungen.

Gemäß der Fig. 1 ist das Gehäuse 10 im Bereich des Fußlängsgewölbes angeordnet, also eines erhabenen Bereichs des Fußes, in dem grundsätzlich keine großen Belastungen einwirken. Gleichwohl sollte das Gehäuse 10 über eine geeignete Mindeststabilität verfügen, um den Fuß auch in diesem Bereich ausreichend abzustützen, gleichzeitig aber die elektronischen Bauelemente ausreichend zu schützen.

Wie man der Schnittansicht gemäß der Fig. 2 entnehmen kann, ist das Gehäuse 10 zweckmäßig korrespondierend zu einer durchschnittlichen Form eines Fußlängsgewölbes ausgebildet. Dies bedingt die in der Fig. 2 abgebildete sanft gewölbte Form in Längsrichtung am hinteren Ende des Gehäuses 10 und eine deutlich stärkere Wölbung am vorderen Ende des Gehäuses 10. In Querrichtung ist das Gehäuse 10 zur Außenkante der Schuheinlage 2 insgesamt keilförmig ausgebildet. Durch die Form des Gehäuses 10 kann das Fußlängsgewölbe auch abgestützt werden, etwa in der Art einer orthopädischen Schuheinlage.

Grundsätzlich kann auch auf der Oberseite des Gehäuses 10, oder entlang seines Randes verlaufend, ein weiterer Kraftsensor 3 vorgesehen sein, der gezielt Fehlbelastungen im Bereich des Fußlängsgewölbes erfassen kann.

Gemäß der Fig. 2 ist in dem abgedichteten Gehäuse 10 eine elektrische Leiterplatte 17 aufgenommen, auf der die Auswertungseinheit 11, die elektrische Energiequelle 13 und die Feedback-Steuerungssignal-Generator 20 sowie ggf. weitere elektronische Bauelemente angeordnet sind. Insbesondere kann auch die Feedback-Ausgabe-Einheit 21 auf der Leiterplatte angeordnet sein. Wenngleich in der Fig. 2 als gesonderte elektronische Bauelemente dargestellt, können sämtliche Funktionen in eine geeignete Prozessoreinheit integriert sein, insbesondere in eine MCU (Microcontrollerunit).

Das Feedback-Steuerungssignal wird an den jeweiligen Aktuator 22 ausgegeben, der als Feedback-Ausgabe-Einheit wirkt, um ein Feedbacksignal im Falle von Fehlbelastungen oder Überlastungen des Fußes zu erzeugen. Im Falle einer normalen Belastung, also wenn die Auswertungseinheit 11 anhand von vorbestimmten Kriterien feststellt, dass keine Fehlbelastungen oder Überlastungen vorliegen, wird von der Feedback-Ausgabe-Einheit zweckmäßig kein Feedbacksignal erzeugt. Dieses wird bevorzugt nur ausgegeben, wenn Fehlbelastungen oder Überlastungen des Fußes durch die Auswertungseinheit 11 bestimmt wurden.

Ein solches Kriterium könnte ein Schwellenwert für das Kraftsignal eines jeweiligen Kraftsensors 3 oder für das vorgenannte gemittelte Kraftsignal sein. Wenn mehrere Kraftsensoren 3 vorgesehen sind, können auch mehrere Schwellenwerte vorgegeben sein, die auch verschieden zueinander sein können. Diese Schwellenwerte können fest vorgegeben sein, können jedoch auch durch die vorgenannte Programmierung über die I/O-Schnittstelle 12 geändert werden. Beispielsweise können Bewegungsprofile über die I/O-Schnittstelle 12 ausgelesen und extern beurteilt und ausgewertet werden, etwa auf dem Computer eines Physiotherapeuten. Dieser kann anhand einer geeigneten Software für den jeweiligen Patienten geeignete Schwellenwerte festlegen.

Bevorzugt ist der Aktuator 22 auf der Oberseite des Gehäuses 10 angeordnet, was insbesondere dann von Vorteil ist, wenn der Aktuator 22 Vibrationen oder Verstellbewegungen unmittelbar auf die Oberseite der Schuheinlage 2 und damit auf das Fußlängsgewölbe einer Person übertragen soll, weil auf diese Weise ein Feedback-Signal taktil unmittelbar auf die Person übertragen werden kann. Es hat sich gezeigt, dass die Unterseite des Fußlängsgewölbes ein sehr empfindlicher, sensibler Bereich ist, sodass auch geringe Signalstärken von der Person wahrgenommen werden können. Somit kann ein geringer Energieverbrauch gewährleistet werden.

Selbstverständlich kann der Aktuator 22 auch an beliebigen anderen Bereichen der Schuheinlage 2 vorgesehen sein, insbesondere auf der Oberseite oder unmittelbar unterhalb eines Stoffbezugs der Schuheinlage 2. Weitere Positionen können grundsätzlich sein: der Bereich seitlich neben dem Fußlängsgewölbe, also dort wo der Fuß mit relativ hoher Gewichtskraft auf der Schuheinlage abgestützt ist; der Fersenbereich der Schuheinlage, beispielsweise seitlich oder zwischen den beiden Kraftsensoren 3 im Fersenbereich (vgl. Fig. 1); der Ballenbereich der Schuheinlage 1, beispielsweise seitlich oder zwischen den Kraftsensoren 3 im Ballenbereich (vgl. Fig. 1). Dabei wird bevorzugt, wenn der jeweilige Aktuator nicht mit einem Kraftsensor 3 überlappt, um eine hohe Messgenauigkeit zu gewährleisten.

Grundsätzlich können auch mehrere Aktuatoren in der Schuheinlage 2 verteilt angeordnet sein, insbesondere an den vorgenannten Positionen.

Wie in der Fig. 2 dargestellt, kann der Aktuator 22 auch mechanisch mit einem Übertragungselement 23 gekoppelt sein, um taktile Reize gezielt auf einen gewünschten Bereich auf der Oberseite der Schuheinlage 2 zu übertragen. Beispielsweise kann das Übertragungselement 23 in einem Ruhezustand, also einem nicht aktivierten Zustand, nicht über die Oberseite des Gehäuses 10 hinausragen, jedoch in einem aktivierten Zustand, also einem Zustand mit taktiler Enervation der Fußunterseite, über die Oberseite des Gehäuses 10 hinausragen, also zur taktilen Reizübertragung auf die Fußunterseite eine Hin- und Herbewegung ausführen.

Zur taktilen Reizübertragung kann jedoch auch grundsätzlich ausreichend sein, wenn eine Vibration des Gehäuses 10 oder dessen der Schuheinlage 2 zugewandten Oberseite erzeugt wird. Hierzu können ein Ober- und Unterteil des Gehäuses relativ zueinander verstellbar gelagert sein.

Als mögliche Position des Aktuators 22 kann auch eine Position direkt unter dem oberen Gehäuseteil gewählt werden, sodass der taktile Reiz unmittelbar auf das Gehäuse 10 übertragen wird, insbesondere direkt an dessen Oberteil.

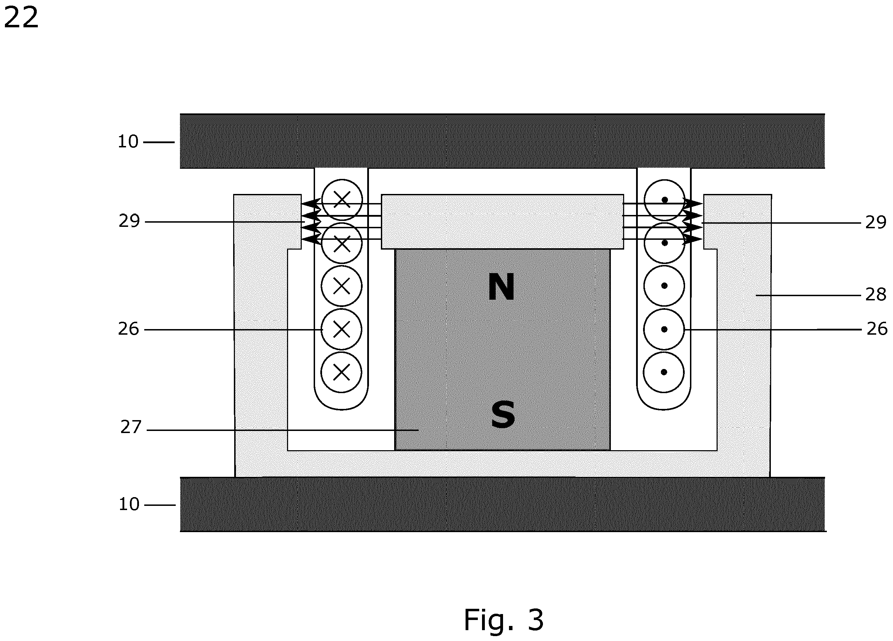

In Fig. 3 ist eine Ausführungsform des Aktuators 22 gezeigt, der eine Feedback-Ausgabe-Einheit gemäß einer weiteren Ausführungsform der vorliegenden Erfindung darstellt. Dieser Aktuator 22 umfasst einen Permanentmagneten 27, der mittig angeordnet ist, sowie ein weichmagnetisches Führungsmaterial 28, das im äußeren Bereich des Aktuators 22 angeordnet ist und zwei symmetrisch zu einer Mittellinie des Aktuators 22 angeordnete Erreger-Spulen Anordnungen 26, die an einer Oberseite des abgedichteten Gehäuses 10 befestigt sind und in entsprechende Öffnungen des Aktuators eintauchen. Der Aktuator 22 ist also analog zu einem Lautsprecher aufgebaut, wobei der Permanentmagnet 27 zusammen mit dem weichmagnetischen Führungsmaterial 28 zur Führung des magnetischen Flusses mit dem unteren Teil des abgedichteten Gehäuses 10 verbunden ist. Wenn ein sich ändernder Strom durch die Erreger-Spule 26 fließt, üben Erreger-Spule 26 und Permanentmagnet 27 eine Kraft entlang der magnetischen Feldlinien 29 aus, die auf das Längsgewölbe des Fußes über die Gehäuseoberseite 10 übertragen wird. Somit wird ein Bio-Feedback auf den Fuß übertragen.

Grundsätzlich ist es möglich, das gleiche Prinzip in umgekehrter Weise auch als Kraftsensor 3 zu verwenden, wobei bei dieser Funktionalität die Bewegung des Fußes die Spulen nach unten drückt, wodurch ein Strom in den Spulen induziert wird, der als Messsignal dienen kann. Durch diese Anordnung ist wahlweise eine Verwendung des Elements aus Fig. 3 als Aktuator oder Kraftsensor 3 möglich.

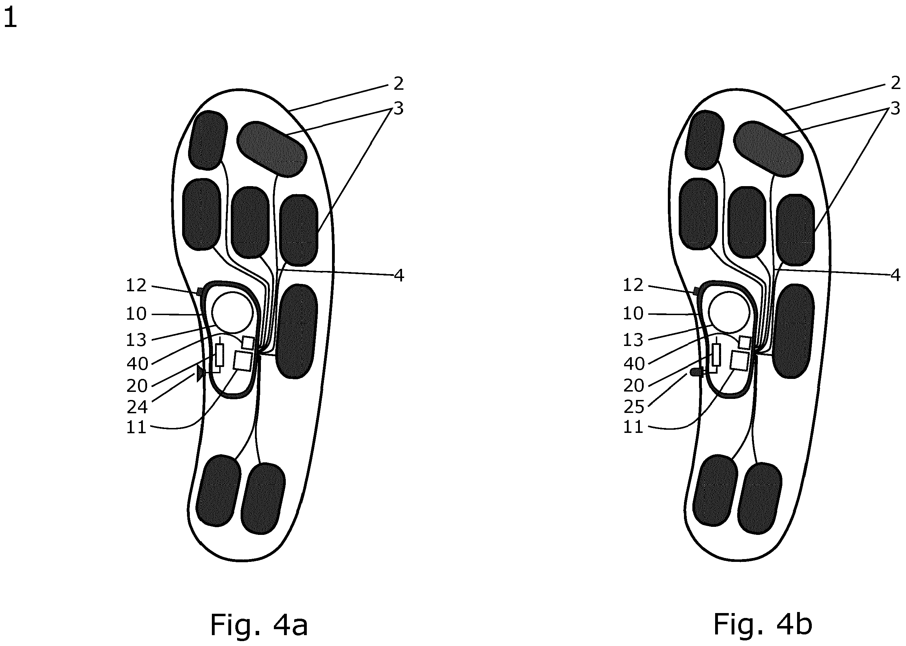

In den Figuren 4a und 4b sind weitere Ausführungsformen der vorliegenden Erfindung dargestellt. Fig. 4a zeigt dabei eine Ausführungsform, bei der der Feedback-Steuerungssignal-Generator 20 mit einem akustischen Signalgeber 24 als Feedback-Ausgabe-Einheit 21 verbunden ist, die das Feedback-Steuerungssignal empfängt und ein akustisches Signal zur Überwachung von Belastungen und/oder Teilbelastungen des Fußes ausgibt. Der akustische Signalgeber 24 kann dabei bevorzugt als einfacher Lautsprecher vorliegen und ist beispielsweise am Randbereich der Schuheinlage 2 oder in bevorzugter Weise außerhalb des Schuhs befestigt, so dass das akustische Signal für den Benutzer hörbar ist. Fig. 4b zeigt dabei eine weitere Ausführungsform, bei der der Feedback-Steuerungssignal-Generator 20 mit einem optischen Signalgeber bzw. Leuchtelement 25 als Feedback-Ausgabe-Einheit 21 verbunden ist, die das Feedback-Steuerungssignal empfängt und ein Leuchtsignal zur Überwachung von Belastungen und/oder Teilbelastungen des Fußes ausgibt. Das Leuchtelement 25 kann insbesondere als LED vorliegen und ist bevorzugter Weise im vorderen Außenbereich des Schuhs angeordnet. Hierzu ist eine geeignete Kabelverbindung zwischen Schuheinlage 2 und Schuhaußenseite erforderlich, auf der der optische Signalgeber bzw. das Leuchtelement 25 angeordnet ist.

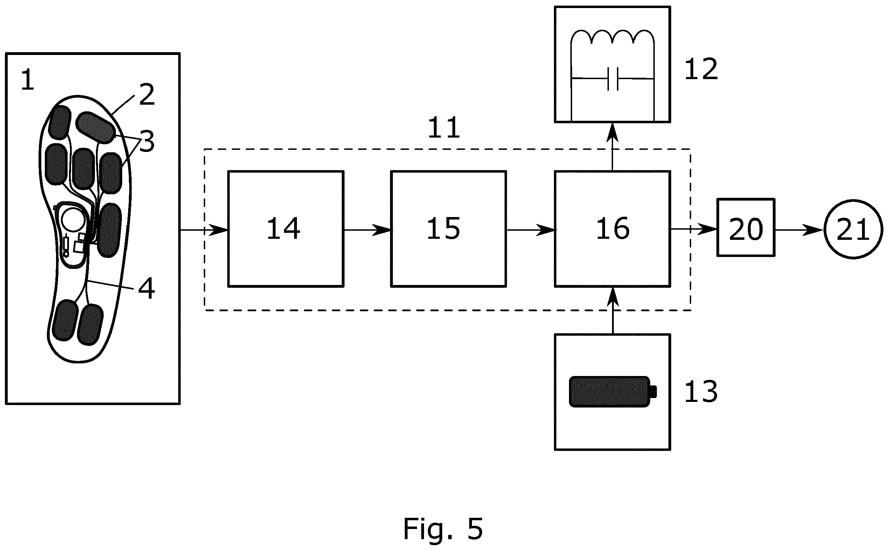

In Fig. 5 ist ein Blockschaltbild eines Ausführungsbeispiels der vorliegenden Erfindung dargestellt. Hierbei messen zunächst die Sensoren 3 der Vorrichtung 1 die durch den Fuß erzeugten Kräfte und leiten dann eine Mehrzahl von analogen Messsignalen zunächst an jeweilige Eingänge eines Multiplexers 14 weiter, der die jeweiligen Signale dann an einen Analog/Digital-Wandler 15 weiterleitet, der diese in digitale Signale umwandelt und an einen Microcontroller (MCU) 16 weiterleitet, der diese weiterverarbeitet. Die zuvor genannten Einheiten 14, 15 und 16 bilden zumindest ein Teil der Auswertungseinheit 11 aus. Des Weiteren kann die MCU 16 mit Signalen aus der I/O-Schnittstelle 12 interagieren.
Das gesamte elektronische System wird durch die elektrische Energiequelle 13 mit Strom versorgt. Nach geeigneter Auswertung der Messsignale, wie vorstehend beschrieben, wird der Feedback-Steuerungssignal-Generator 20 angesteuert, um ein Feedback-Steuerungssignal zu erzeugen, das an die Feedbacksignal-Ausgabeeinheit 21 weitergeleitet wird, die daraufhin ein Feedback ausgibt, insbesondere ein taktiles Feedback, wie vorstehend beschrieben.

Durch die genannte Anordnung können bereits im Handel erhältliche Foliensensoren in der Vorrichtung 1 als Kraftsensoren verwendet werden, wobei zweckmäßig ein periodisches Abtasten der Kraftsensoren erfolgt, um die Messsignale auszulesen und anschließend weiter zu verarbeiten, wie vorstehend ausgeführt. Zum Auslesen der Messsignale kann eine Linearisierung der Sensorkennlinien erforderlich sein. Grundsätzlich kann eine solche Linearisierung jedoch auch anhand der ausgelesenen Messsignale in dem MCU 20 vorgenommen werden. Des Weiteren kann zur Auswertung der Messsignale über eine Summenbildung ein Belastungs-Mittelwert gebildet werden, beispielsweise über sämtliche Kraftsensoren 3 der Schuheinlage 2 oder auch über Teilgruppen hiervon, wie vorstehend beschrieben. Bei der Überschreitung eines vorher bestimmten Schwellenwertes kann der Aktuator gesteuert werden, um ein Feedbacksignal zu erzeugen. Die Schwellenwerte können über die I/O-Schnittstelle 12 in der MCU gesetzt werden und werden üblicherweise als Parameter in der MCU gespeichert.

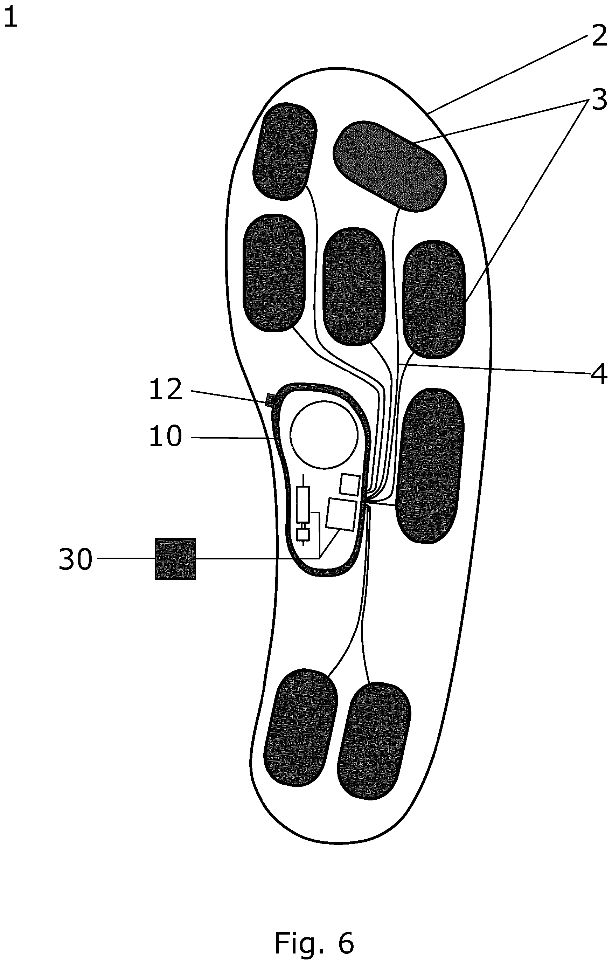

In Fig. 6 ist eine weitere mögliche Ausführungsform der erfindungsgemäßen Vorrichtung 1 dargestellt, bei der zusätzlich zur taktilen Feedback-Ausgabe eine Übertragungs-Einheit 30 angeordnet ist, mit der die Vorrichtung 1 Daten mit einer externen Anzeige-Einheit, wie z.B. einem Smartphone oder einer Display-Einheit austauschen kann. Insbesondere kann hierdurch der aktuelle Zustand der Fußbelastungen angezeigt werden. Die Übertragungs-Einheit 30 kann insbesondere als Kabel, oder in bevorzugter Weise als Funksender vorliegen, wobei der Funksender jedoch außerhalb des Schuhes angeordnet sein muss, um eine Signalübertragung zu gewährleisten. Falls der Funksender fälschlicher Weise direkt an oder unter der Schuheinlage befestigt wird, so kann der Fuß den Funksender abschirmen und einen zuverlässigen Empfang stören oder sogar komplett verhindern.

### Bezugszeichenliste

- 1: Vorrichtung zur Vermeidung von übermäßigen Belastungen auf den menschlichen Fuß beim Gehen
- 2: Schuheinlage
- 3: Kraftsensor(en)
- 4: Elektrische Leitungen

- 10: Abgedichtetes Gehäuse
- 11: Auswertungseinheit oder CPU
- 12: I/O-Schnittstelle (Steckverbindung oder NFC-Induktionsspule)
- 13: Elektrische Energiequelle
- 14: Multiplexer
- 15: Analog-Digitalkonverter
- 16: Microcontroller (MCU)
- 17: Elektrische Leiterplatte

- 20: Feedback-Steuerungssignal-Generator- (Endstufe)
- 21: Feedback-Ausgabe-Einheit
- 22: Aktuator
- 23: Übertragungselement
- 24: Akustischer Signalgeber
- 25: Optischer Signalgeber bzw. Leuchtelement
- 26: Erreger-Spule
- 27: Permanentmagnet
- 28: Weichmagnetisches Führungsmaterial
- 29: Magnetische Feldlinien
- 30: Übertragungs-Einheit
- 31: Externer Funksensor
- 32: Kabel

- 40: Speicherelement

## Patentansprüche

1. Vorrichtung (1) zur Vermeidung von übermäßigen Belastungen auf den menschlichen Fuß beim Gehen, umfassend:
eine Schuheinlage (2) mit mindestens einem Kraftsensor (3) zum Erzeugen eines Messsignals, das Belastungen und/oder Teilbelastungen des Fußes beim Gehen anzeigt;
eine Auswertungseinheit (11), die elektrisch leitend mit dem mindestens einen Kraftsensor (3) verbunden ist und ausgelegt ist, um das Messsignal von dem mindestens einen Kraftsensor (3) auszuwerten;
eine elektrische Energiequelle (13), die ausgelegt ist, um Strom für die Auswertungseinheit (11) bereitzustellen; und
einen Feedback-Steuerungssignal-Generator (20) mit einer zugeordneten Feedback-Ausgabe-Einheit (21); wobei
der Feedback-Steuerungssignal-Generator (20) ausgelegt ist, um auf Grundlage der Auswertung durch die Auswertungseinheit (11) ein Feedback-Steuerungssignal zu erzeugen,
die mindestens eine Feedback-Ausgabe-Einheit (21) ausgelegt ist, um durch das Feedback-Steuerungssignal gesteuert zu werden, um ein Feedbacksignal aufgrund von Belastungen und/oder Teilbelastungen des Fußes auszugeben, und
die Feedback-Ausgabe-Einheit als Aktuator (22) ausgebildet ist, der das Feedback-Steuerungssignal erhält und ausgelegt ist, um ein taktiles Feedback im Bereich des Fuß-Längsgewölbes auszugeben;
wobei die Auswertungseinheit (11) und die Energiequelle (13) als gemeinsame Einheit in einem Hohlraum eines Gehäuses (10) angeordnet sind, das in die Schuheinlage (2) im Bereich seines Fuß-Längsgewölbes integriert ist,
**dadurch gekennzeichnet, dass** der Feedback-Steuerungssignal-Generator (20) ebenfalls als Bestandteil der gemeinsamen Einheit in dem Hohlraum des Gehäuses angeordnet ist,
dass das Gehäuse (10) gewölbt und korrespondierend zu einer durchschnittlichen Form eines Fuß-Längsgewölbes ausgebildet ist, um einen Fuß im Bereich des Fuß-Längsgewölbes unmittelbar abzustützen, und dass der Aktuator (22) auf dem oder unmittelbar an dem Gehäuse (10) oder in dem Gehäuse (10) angeordnet ist, um das taktile Feedback im Bereich des Fuß-Längsgewölbes auszugeben.

2. Vorrichtung (1) nach Anspruch 1, wobei das Gehäuse (10) eine sanft gewölbte Form in Längsrichtung am hinteren Ende des Gehäuses (10) und eine stärkere Wölbung am vorderen Ende des Gehäuses (10) aufweist und wobei das Gehäuse in Querrichtung zur Außenkante der Schuheinlage (2) keilförmig ausgebildet.

3. Vorrichtung (1) nach Anspruch 1 oder 2, wobei der Aktuator (22) ausgelegt ist, um als taktiles Feedback Vibrationen auf dem Gehäuse oder in dessen unmittelbarer Nähe zu erzeugen.

4. Vorrichtung (1) nach Anspruch 1 oder 2, wobei der Aktuator (22) umfasst:
einen Permanentmagneten (27), der mittig angeordnet ist,
ein weichmagnetisches Führungsmaterial (28), das im äußeren Bereich des Aktuators (22) angeordnet ist, und
eine symmetrisch um die Mittellinie des Aktuators (22) angeordnete Erreger-Spule (26), die mit einer Platte (10) verbunden ist, die auf der Oberseite der Schuheinlage (2) oder in deren unmittelbarer Nähe angeordnet ist, wobei
die Erreger-Spule (26) in eine entsprechende Öffnung des Aktuators (22) eintaucht, um im Wesentlichen senkrecht zu den magnetischen Feldlinien (29) des Permanentmagneten (27) zu stehen, und ausgelegt ist, um durch Erzeugen eines Stromflusses in der Erreger-Spule (26) das taktile Feedback zu erzeugen.

5. Vorrichtung nach einem der vorherigen Ansprüche, mit einer Mehrzahl von Kraftsensoren (3), die verteilt in oder auf der Schuheinlage (2) angeordnet sind.

6. Vorrichtung nach Anspruch 5, wobei die Auswertungseinheit (11) ausgelegt ist, um Messsignale von mehreren Kraftsensoren (3) aufzusummieren und auf Grundlage eines Mittel- oder Summenwerts das Feedback-Steuerungssignal zu erzeugen.

7. Vorrichtung (1) nach einem der Ansprüche 1 bis 5, wobei die Vorrichtung eine Mehrzahl von Kraftsensoren (3) und eine Mehrzahl von Aktuatoren (22) umfasst, die verteilt in oder auf der Schuheinlage (2) angeordnet sind und ausgelegt sind, um an mehreren Positionen der Schuheinlage (2) ein taktiles Feedback zu erzeugen.

8. Vorrichtung (1) nach einem der vorherigen Ansprüche, weiterhin umfassend
eine Feedback-Ausgabe-Einheit (21), die ein akustischer Signalgeber (24) ist, der ausgelegt ist, um das Feedback-Steuerungssignal zu empfangen und ein akustisches Signal als Feedback auszugeben; und/oder
eine Feedback-Ausgabe-Einheit (21), die ein Leuchtelement (25) ist, welches ausgelegt ist, um das Feedback-Steuerungssignal zu empfangen und ein optisches Signal als Feedbacksignal auszugeben.

9. Vorrichtung (1) nach einem der vorherigen Ansprüche, wobei das Gehäuse eine elektrische Leiterplatte (17) aufweist und die Auswertungseinheit (11), die elektrische Energiequelle (13) und der Feedback-Steuerungssignal-Generator (20) in dem Gehäuse (10) angeordnet sind.

10. Vorrichtung (1) nach einem der vorherigen Ansprüche, weiterhin umfassend mindestens eine I/O-Schnittstelle (12) zum Auslesen und/oder Programmieren der Vorrichtung (1), wobei die I/O-Schnittstelle (12) insbesondere als NFC-Schnittstelle ausgebildet ist.

11. Vorrichtung (1) nach einem der vorherigen Ansprüche, weiterhin umfassend ein Speicherelement (40) zum Speichern von Daten der Auswertungseinheit (11).

12. Vorrichtung (1) nach einem der vorherigen Ansprüche, weiterhin umfassend eine Übertragungs-Einheit (30) um Daten und/oder Signale der Auswertungseinheit (11) oder das Feedback-Steuerungssignal des Feedback-Steuerungssignal-Generators (20) an eine externe Anzeige-Einheit zu übertragen.

13. Vorrichtung (1) nach Anspruch 12, wobei die Übertragungs-Einheit (30) ein Funksensor ist, der extern zur Schuheinlage (2) vorgesehen ist.

14. Schuh, umfassend eine Vorrichtung (1) nach einem der vorhergehenden Ansprüche.

15. Verfahren zum Betreiben der Vorrichtung (1) nach einem der vorhergehenden Ansprüche, mit den folgenden Schritten:
Messen von Belastungen und/oder Teilbelastungen mit dem mindestens einen Kraftsensor (3) und Erzeugen eines Messsignals, das die Belastungen und/oder Teilbelastungen des Fußes anzeigt;
Auswerten des durch die Auswertungseinheit (11) empfangenen Messsignals und Erzeugen eines Feedback-Steuerungssignals auf Grundlage der Auswertung durch die Auswertungseinheit (11) und Steuern der mindestens einen Feedback-Ausgabe-Einheit (21), um ein Feedbacksignal aufgrund von Belastungen und/oder Teilbelastungen des Fußes auszugeben; und
Ausgeben des Feedbacksignals durch die mindestens eine Feedback-Ausgabe-Einheit (21);
wobei das Gehäuse gewölbt und korrespondierend zu einer durchschnittlichen Form eines Fuß-Längsgewölbes ausgebildet ist und einen Fuß im Bereich des Fuß-Längsgewölbes unmittelbar abstützt, und
wobei der Aktuator (22) auf dem oder unmittelbar an dem Gehäuse (10) oder in dem Gehäuse (10) angeordnet ist, das Feedback-Steuerungssignal erhält und ein taktiles Feedback im Bereich des Fuß-Längsgewölbes und auf dem Gehäuse oder in dessen unmittelbarer Nähe ausgibt.

## Claims

1. A device (1) for avoiding excessive loads on the human foot when walking, comprising: a
a shoe insole (2) having at least one force sensor (3) for generating a measurement signal which indicates loads and/or partial loads on the foot when walking;
an analyzing unit (11) electrically connected to the at least one force sensor (3) and configured for analyzing the measurement signal of the at least one force sensor (3);
an electric power source (13) configured for providing electric power to the analyzing unit (11); and
a feedback control signal generator (20) having at least one feedback output unit (21) associated therewith; wherein
the feedback control signal generator (20) is configured to generate a feedback control signal on the basis of the analysis by the analyzing unit (11),
the at least one feedback output unit (21) is configured to be controlled by the feedback control signal to output a feedback signal due to loads and/or partial loads on the foot, and
the at least one feedback output unit is an actuator (22), which receives the feedback control signal and is configured to output a tactile feedback in the region of the foot longitudinal arch;
wherein the analyzing unit (11) and the energy source (13) are disposed as a common unit in a cavity of a housing (10) which is integrated into the shoe insole (2) in the region of its foot longitudinal arch,
**characterized in that** the feedback control signal generator (20) is also disposed as a part of the common unit in the cavity of the housing,
that the housing (10) is curved shape and formed corresponding to an average shape of a foot longitudinal arch to directly support a foot in the region of the foot longitudinal arch, and
that the actuator (22) is disposed on the housing (10) or directly at the housing (10) or in the housing (10) to output the tactile feedback in the region of the foot longitudinal arch.

2. The device (1) as claimed in claim 1, wherein the housing (1) has a gentle curved shape lengthwise at the rear end of the housing (10) and a much stronger curvature at the front end of housing (10) and wherein the housing (10) is generally wedge-shaped in the transverse direction toward the outer edge of shoe insole (2).

3. The device (1) as claimed in claim 1 or claim 2, wherein the actuator (22) is configured for generating, as the tactile feedback, vibrations on the housing or in the immediate vicinity of the housing.

4. The device (1) as claimed in claim 1 or 2, said actuator (22) comprising:
a permanent magnet (27) disposed at a central position,
a soft magnetic guiding material (28) disposed in the outer region of the actuator (22), and
an excitation coil (26) arranged symmetrically around the centerline of the actuator (22) and connected to a plate (10) arranged on the upper side of the shoe insole (2) or in its immediate vicinity, wherein
the excitation coil (26) is immersed in a corresponding opening of the actuator (22) to be substantially perpendicular to the magnetic field lines (29) of the permanent magnet (27), for generating the tactile feedback by generating a current flow in the excitation coil (26).

5. The device (1) as claimed in any of the preceding claims, comprising a plurality of force sensors (3) distributed in or on the shoe insole (2).

6. The device (1) as claimed in claim 5, wherein the analyzing unit (11) is configured for summing up measurement signals of a plurality of force sensors (3) and generating the feedback control signal on the basis of a mean value or sum value.

7. The device (1) as claimed in any of claims 1 to 5, said device comprising a plurality of force sensors (3) and a plurality of actuators (22) distributed in or on the shoe insole (2) and configured for providing tactile feedback at a plurality of positions of the shoe insole (2).

8. The device (1) as claimed in any of the preceding claims, further comprising
a feedback output unit (21) which is an acoustic signal generator (24) and configured for receiving the feedback control signal and outputting an acoustic signal as feedback; and/or
a feedback output unit (21), which is a light emitting element (25) and configured for receiving the feedback control signal and outputting an optical signal as a feedback signal.

9. The device (1) as claimed in any of the preceding claims, wherein the housing comprises an electrical circuit board (17) and the analyzing unit (11), the electric power source (13) and the feedback control signal generator (20) are disposed in the housing (10).

10. The device (1) as claimed in any of the preceding claims, wherein further comprising at least one I/O interface (12) for reading out and/or programming the device (1), the I/O interface (12) being designed in particular as an NFC interface.

11. The device (1) as claimed in any of the preceding claims, further comprising a memory element (40) for storing data of the analyzing unit (11).

12. The device (1) as claimed in any of the preceding claims, further comprising a transmission unit (30) for transmitting data and/or signals of the analyzing unit (11) or the feedback control signal of the feedback control signal generator (20) to an external display unit.

13. The device (1) as claimed in claim 12, wherein the transmission unit (30) is an RF sensor provided externally to the shoe insole (2).

14. A shoe, comprising a device (1) as claimed in any of the preceding claims.

15. A method of operating the device (1) as claimed in any of the preceding claims, comprising the steps:
measuring loads and/or partial loads using the at least one force sensor (3) and generating a measurement signal indicating the loads and/or partial loads on the foot;
analyzing the measurement signal received by the analyzing unit (11) and generating a feedback control signal on the basis of the analysis by the analyzing unit (11) and controlling the at least one feedback output unit (21) to output a feedback signal due to loads and/or partial loads on the foot; and
outputting the feedback signal via the at least one feedback output unit (21); wherein
the housing directly supports the foot in the region of the foot longitudinal arch, and
the actuator (22) receives the feedback control signal and outputs a tactile feedback in the region of the foot longitudinal arch and on the housing or in the immediate vicinity of the housing;
wherein the housing (10) is curved shape and formed corresponding to an average shape of a foot longitudinal arch and directly supports a foot in the region of the foot longitudinal arch, and
the actuator (22) is disposed on the housing (10) or directly at the housing (10) or in the housing (10), receives the feedback control signal and outputs a tactile feedback in the region of the foot longitudinal arch and on the housing or in the immediate vicinity of the housing.

## Revendications

1. Un dispositif (1) permettant d'éviter des appuis excessive sur le pied humain lors de la marche, comprenant
un insert intérieure de la chaussure (2) avec au moins un capteur de force (3) pour générer un signal de mesure indiquant les charges et/ou les charges partielles sur le pied pendant la marche ;
une unité d'évaluation (11) qui est reliée de manière électriquement conductrice à au moins un capteur de force (3) et qui est conçue pour évaluer le signal de mesure provenant d'au moins un capteur de force (3) ;
une source d'énergie électrique (13) conçue pour alimenter l'unité d'évaluation (11)
un générateur de signaux de contrôle de rétroaction (20) ayant une unité de sortie de rétroaction associée (21) ; dans lequel
le générateur de signaux de contrôle de rétroaction (20) est adapté pour générer un signal de contrôle de rétroaction basé sur l'évaluation par l'unité d'évaluation (11),
la ou les unités de sortie de rétroaction (21) sont adaptées pour être contrôlées par le signal de contrôle de rétroaction afin d'émettre un signal de rétroaction en raison de charges et/ou de charges partielles sur le pied, et
l'unité de sortie de rétroaction est formée d'un actionneur (22) qui reçoit le signal de contrôle de rétroaction et est conçu pour produire une rétroaction tactile dans la zone de la voûte longitudinale du pied ,
dans lequel l'unité d'évaluation (11) et la source d'énergie (13) sont disposées comme une unité commune dans une cavité d'un boîtier (10) qui est intégré dans l'insert de la chaussure (2) dans la région de sa voûte longitudinale du pied,
**caractérisé en ce que** le générateur de signaux de contrôle de rétroaction (20) est également disposé comme un composant de l'unité commune dans la cavité du boîtier,
**que** le boîtier (10) est incurvé et correspond à une forme moyenne d'une voûte longitudinale du pied afin de soutenir directement un pied dans la zone de la voûte longitudinale du pied, et
**en ce que** l'actionneur (22) est disposé sur ou directement sur le boîtier (10) ou dans le boîtier (10) afin d'émettre la rétroaction tactile dans la région de la voûte longitudinale du pied.

2. Le dispositif (1) selon la revendication 1, dans lequel le boîtier (10) a une forme légèrement incurvée dans la direction longitudinale à l'extrémité arrière du boîtier (10) et une courbure plus forte à l'extrémité avant du boîtier (10) et dans lequel le boîtier est en forme de coin dans la direction transversale par rapport au bord extérieur de l'insert de la chaussure (2).

3. Le dispositif (1) selon la revendication 1 ou 2, dans lequel l'actionneur (22) est conçu pour générer des vibrations sur le boîtier ou dans son voisinage immédiat en tant que retour tactile.

4. Le dispositif (1) selon la revendication 1 ou 2, dans lequel l'actionneur (22) comprend
un aimant permanent (27) disposé au centre,
un matériau de guidage magnétique doux (28) disposé dans la région extérieure de l'actionneur (22), et
une bobine d'excitation (26) disposée symétriquement par rapport à l'axe central de l'actionneur (22) et reliée à une plaque (10) disposée sur la face supérieure de l'insert de la chaussure (2) ou à proximité immédiate de celle-ci, dans laquelle
la bobine d'excitation (26) est immergée dans une ouverture correspondante de l'actionneur (22) de manière à être sensiblement perpendiculaire aux lignes de champ magnétique (29) de l'aimant permanent (27) et est adaptée pour fournir la rétroaction tactile en générant un flux de courant dans la bobine d'excitation (26).

5. Le dispositif selon quelconque des revendications précédentes, comprenant une pluralité de capteurs de force (3) répartis dans ou sur l'insert intérieure de la chaussure (2).

6. Le dispositif selon la revendication 5, dans lequel l'unité d'évaluation (11) est conçue pour additionner les signaux de mesure de plusieurs capteurs de force (3) et pour générer le signal de contrôle de rétroaction sur la base d'une valeur moyenne ou d'une somme.

7. Le dispositif (1) selon quelconque des revendications 1 à 5, ledit dispositif comprenant une pluralité de capteurs de force (3) et une pluralité d'actionneurs (22) répartis dans ou sur l'insert de chaussure (2) et adaptés pour générer un retour tactile à plusieurs positions de l'insert de chaussure (2) .

8. Le dispositif (1) selon quelconque des revendications précédentes, comprenant en outre
une unité de sortie de rétroaction (21) qui est un générateur de signaux acoustiques (24) adapté pour recevoir le signal de contrôle de rétroaction et émettre un signal acoustique en guise de rétroaction ; et/ou
une unité de sortie de rétroaction (21) qui est un élément d'éclairage (25) adapté pour recevoir le signal de contrôle de rétroaction et pour émettre un signal optique en tant que signal de rétroaction.

9. Le dispositif (1) selon quelconque des revendications précédentes, dans lequel le boîtier comprend une carte de circuit électrique (17) et l'unité d'évaluation (11), la source d'énergie électrique (13) et le générateur de signaux de contrôle de rétroaction (20) sont disposés dans le boîtier (10) .

10. Le dispositif (1) selon quelconque des revendications précédentes, comprenant en outre au moins une interface E/S (12) pour la lecture et/ou la programmation du dispositif (1), l'interface E/S (12) étant conçue en particulier comme une interface NFC.

11. Le dispositif (1) selon quelconque des revendications précédentes, comprenant en outre un élément de mémoire (40) pour le stockage des données de l'unité d'évaluation (11).

12. Le dispositif (1) selon quelconque des revendications précédentes, comprenant en outre une unité de transmission (30) pour transmettre des données et/ou des signaux de l'unité d'évaluation (11) ou le signal de contrôle de rétroaction du générateur de signaux de contrôle de rétroaction (20) à une unité d'affichage externe.

13. Le dispositif (1) selon la revendication 12, dans lequel l'unité de transmission (30) est un capteur radio prévu à l'extérieur d'insert intérieure de la chaussure (2).

14. Une chaussure comprenant un dispositif (1) selon quelconque des revendications précédentes.

15. Un procédé fonctionnement le dispositif (1) selon quelconque des revendications précédentes, comprenant les étapes suivantes :
mesurer des charges et/ou des charges partielles avec au moins un capteur de force (3) et générer un signal de mesure indiquant les charges et/ou les charges partielles sur le pied ;
évaluer le signal de mesure reçu par l'unité d'évaluation (11) et générer un signal de contrôle de rétroaction basé sur l'évaluation par l'unité d'évaluation (11) et commander la au moins une unité de sortie de rétroaction (21) pour qu'elle émette un signal de rétroaction basé sur les charges et/ou les charges partielles sur le pied ; et
sortir le signal de rétroaction par au moins une unité de sortie de rétroaction (21) ;
dans lequel le boîtier est incurvé et formé de manière à correspondre à une forme moyenne d'une voûte longitudinale du pied et soutient directement un pied dans la zone de la voûte longitudinale du pied, et
dans lequel l'actionneur (22) est disposé sur ou directement sur le boîtier (10) ou dans le boîtier (10), reçoit le signal de contrôle de rétroaction et émet une rétroaction tactile dans la région de la voûte longitudinale du pied et sur le boîtier ou à proximité immédiate de celui-ci.
